# EUROPÄISCHE PATENTSCHRIFT

(11) **EP 0 617 789 B1**
(45) Veröffentlichungstag und Bekanntmachung des Hinweises auf die Patenterteilung: **12.03.1997**
(21) Anmeldenummer: 93901706.7
(22) Anmeldetag: 18.12.1992
(51) Int. Cl.: G01N 1/20, A01J 5/04

(54) **VERFAHREN UND VORRICHTUNG ZUR ENTNAHME EINER REPRÄSENTATIVEN MILCHPROBE**
PROCESS AND DEVICE FOR TAKING REPRESENTATIVE MILK SAMPLES
PROCEDE ET DISPOSITIF DE PRELEVEMENT D'UN ECHANTILLON REPRESENTATIF DE LAIT

(30) Priorität: 19.12.1991 DE 4142098
(43) Veröffentlichungstag der Anmeldung: 05.10.1994
(73) Patentinhaber: ULTRAKUST electronic GmbH, D-94239 Gotteszell (DE)
(72) Erfinder: BÖHM, Alfred, D-8374 Viechtach (DE); VAITH, Werner, D-8371 Patersdorf (DE); PENZKOFER, Klaus, D-8375 Ruhmannsfelden (DE); GEISSLER, Andreas, D-8360 Deggendorf (DE); LERACH, Dieter, D-8372 Zwiesel (DE)
(74) Vertreter: Heim, Hans-Karl, Dipl.-Ing.
(86) Internationale Anmeldenummer: EP9202953
(87) Internationale Veröffentlichungsnummer: WO9312413

(56) Entgegenhaltungen:
- EP-A- 0 460 359
- WO-A-82/04127
- DE-A- 3 502 858
- US-A- 4 091 675

## Beschreibung

Die Erfindung betrifft ein Verfahren zur Entnahme einer repräsentativen Milchprobe gemäß Oberbegriff des Anspruchs 1 und eine Vorrichtung, insbesondere zur Durchführung dieses Verfahrens gemäß Oberbegriff des Anspruchs 5.

Ein wesentlicher Aspekt bei einem derartigen Verfahren und einer derartigen Vorrichtung besteht darin, ein repräsentatives und gegebenenfalls reproduzierbares Soll-Volumen für die zu entnehmende Milchprobe zu gewährleisten. Hierzu ist unter anderem die Kenntnis der Liefer-Milchmenge des jeweiligen Lieferanten erforderlich und diese Liefer-Milchmenge wird als eine der Soll-Daten in die Datenerfassungseinrichtung eingegeben, so daß die für die Aufnahme der Milchprobe bestimmte Probeflasche aufgrund dieses Parameters mehr oder weniger konstant gefüllt wird.

Da es das Bestreben ist, eine repräsentative Milchprobe für und über die gesamte gelieferte Milchcharge zu entnehmen, muß der Entnahmevorgang für die Milchprobe in Abhängigkeit vom Gesamtvolumen der Milchcharge steuerbar sein. Hierbei versteht man unter dem Begriff einer repräsentativen Milchprobe den Sachverhalt, daß einerseits nur ein maximales Volumen in der für die Milchprobe bestimmten Probeflasche zur Verfügung steht, andererseits aber die Milchprobe nach Möglichkeit in etwa ein konstantes Volumen aufweisen sollte, unabhängig davon, ob eine volumenmäßig geringe Milchcharge oder eine sehr große Milchcharge gefördert wird. Das angestrebte konstante Volumen für die Milchprobe resultiert daraus, daß für die Untersuchungen und Tests der Milchprobe bestimmte Mindestmengen erforderlich sind.

Darüber hinaus wird mit der repräsentativen Milchprobe angestrebt, die Milchprobe über den gesamten Förder- und Absaugzyklus der entsprechenden Milchcharge zu ziehen. Da abhängig vom Volumen die Milchchargen mit unterschiedlichen Förderleistungen z.B. aus einem Behälter abgesaugt und in den Milchsammelwagen gepumpt werden und somit auch unterschiedliche Förderzeiten auftreten, ist auch dieser Aspekt im Rahmen einer repräsentativen Milchprobe zu berücksichtigen.

Ein weiterer Aspekt bei der Milchprobenentnahme betrifft die verschleppungsfreie Probenahme, die im wesentlichen dadurch gewährleistet wird, daß die Probennahme im Ansaugweg erfolgt.

Ein Verfahren und eine Vorrichtung der eingangs genannten Art, bei dem bzw. bei der die vorstehend genannten Aspekte wenigstens teilweise berücksichtigt sind, sind aus der DE 35 02 858 Al bekannt. Die Messung des Förderstromes erfolgt hierbei mit einem magnetisch induktiven Durchflußmesser (MID). Vorgesehen ist außerdem ein Luftdetektor, der bei Überschreitung eines gewissen Luftanteils in der Förderleitung die Vorrichtung ausschaltet oder Störung meldet. Ein Leitfähigkeitsschalter dient als Signalgeber für eine Mechanik, mit der ein Auslauftrichter verstellt werden kann, um eine programmgemäße Spülung der Milchleitung zu Beginn einer Milchentnahme durchführen zu können.

Nicht berücksichtigt wird dabei jedoch die Tatsache, daß die geförderte Milchcharge in Abhängigkeit vom Förderzeitpunkt unterschiedlich große Luftbeimengungen enthält. Dies ist insbesondere am Ende der Förderung der Fall, wenn bei fetter Milch der auf der Milch aufschwimmende Rahmanteil abgesaugt wird, der für den Lieferanten ein wichtiges Qualitätsmerkmal darstellt. Aufgrund der Lufteinschlüsse wird dies jedoch nicht in der erwünschten Weise durch diese Art der Probenahme erfaßt.

Außerdem ist die Erzeugung von Lufteinschlüssen in der Milch zum Beispiel stark abhängig von dem jeweiligen, die Milch vor Ort absaugenden Personal. Derartige Fehlerquellen beeinflussen nicht nur stark die Förderleistung und die Förderzeit bei der Übernahme einer Milchcharge aus einem Behälter in den Milchsammelwagen oder einen anderen Behälter, sondern auch die Aussagekraft bei der Probenahme.

Aus der DE 40 18 468 Al ist es ferner bekannt, die Förderleistung einer Peristaltikpumpe für die Probenehmer-Einrichtung durch Berücksichtigung einer Annahmekennlinie für diese Pumpe zu bestimmen.

Eine Durchflußmessung für Milch mit einer verhältnismäßig aufwendigen Vorrichtung ist aus der DE 37 37 607 Al bekannt, bei welcher eine Einlauf- und Meßkammer durch eine Trennwand mit Schlitzen oder Sieben voneinander separiert sind. Der Meßkammer wird kontinuierlich Milch zugeführt, die kontinuierlich über einen im wesentlichen senkrechten Schlitz abfließen kann. In der Meßkammer sind eine Gegenelektrode und eine Vielzahl vertikal angeordneter Meßelektroden vorgesehen, die der Ermittlung der Milchmasse durch eine Bestimmung der spezifischen Dichte des Milch-Luft-Gemisches in Abhängigkeit von dessen Höhe dienen. Das gesamte Meßvolumen in der Meßkammer wird in Schichten unterteilt und in Höhenniveaus mit gleichen gegenseitigen Höhenabständen und gleichem Querschnitt Einzelparameter, wie elektrische Leitfähigkeit, Wärmeleitfähigkeit oder Infrarotabsorptionsfähigkeit, gemessen, und eine Verhältniszahl aus dem Meßwert jedes Höhenniveaus und einem Bezugsmeßwert gebildet. Dabei wird der Bezugsmeßwert am Boden der Meßkammer im Bereich der luftfreien, entgasten Milch gemessen. Zur Bestimmung der Leitfähigkeit werden punktförmige Meßelektroden verwendet. Diese relativ aufwendige Meßeinrichtung gewährleistet jedoch nicht in jedem Falle eine repräsentative Milchprobennahme, insbesondere nicht bei stark aufgeschäumter, fetter Milch am Ende der Förderung.

Ein aus der DE 41 08 138 Al bekanntes Meßsystem zur Messung von Flüssigkeiten, insbesondere von Abwasser, sieht einen magnetisch induktiven Durchflußmesser vor, der bei vollgefülltem Leitungsrohr wie ein normaler MID arbeitet. Wenn sich die Füllhöhe im Rohr verringert, können zusätzliche, in der unteren Hälfte des Rohres installierte Elektroden zur Meßwerterfassung herangezogen werden. Eine Wandströmung und Lufteinschlüsse in der Flüssigkeit, wie es bei Milch unumgänglich ist, werden durch dieses Meßsystem nicht bzw. nicht im erforderlichen Maße berücksichtigt.

Ein Verfahren zur Bemessung eines Vorspülvolumens ist in der DE 39 38 076 Al beschrieben. Das ungeprobte und an einer Abzweigstelle am Probenvorlaufgefäß vorbeigeführte Vorspülvolumen wird über den Abstand zwischen einer Erfassungsstelle, die durch einen Signalgeber gebildet ist, und der Abzweigstelle bemessen. Der Signalgeber reagiert auf die Ankunft der Grenzfläche zwischen der Gas- und Flüssigkeitsströmung und startet eine vorzugebende Vorspülzeit. Das Spülen der Leitung erfolgt zu Beginn der Milchabnahme.

Die Vorrichtung der DE-PS 1 224 522 sieht die Anordnung von wenigstens zwei über den Rohrquerschnitt verteilt angeordneter Entnahmekanülen vor, die vom Beginn bis zum Ende der Durchströmung eine vom Staudruck unabhängige Milchmenge in ein Probenentnahmegefäß abzweigen. Die eigentliche Milchprobe wird am Ende des Liefervorganges und nach einem Mischvorgang aus einem Meßraum des Probenentnahmegefäßes entnommen.

Weiterhin ist aus der DE 35 28 827 A1 eine Volumenmeßanlage für Milchsammelwagen mit einer impulsgesteuerten Probenentnahmevorrichtung bekannt. Unabhängig von der Liefermenge wird ein gleichbleibendes Probenvolumen erreicht, indem eine konstante Impulsanzahl vorgegeben ist, das Volumen pro Impuls aber in Abhängigkeit von der Liefermenge berechnet wird.

Der Erfindung liegt die **Aufgabe** zugrunde, ein Verfahren und eine Vorrichtung zu schaffen, welche mit besonderes einfachen Mitteln eine repräsentative Milchprobe unter den Gegebenheiten der Praxis gewährleisten und eine nahezu fehlerfreie quantitative und qualitative Milchbestimmung ermöglichen.

Gelöst wird diese Aufgabe bei einem gattungsgemäßen Verfahren durch die Merkmale des kennzeichnenden Teils des Anspruchs 1 und bei einer gattungsgemäßen Vorrichtung durch die Merkmale des kennzeichnenden Teils des Anspruchs 5. Vorteilhafte Weiterbildungen der Erfindung sind in den Unteransprüchen angegeben.

Erfindungsgemäß erfolgt die Steuerung der Pumpe der Probenehmer-Einrichtung auf der Grundlage des Ist-Volumens der geförderten Milchmenge. Mit anderen Worten geht in die Steuerung dieser Pumpe die momentan und tatsächlich geförderte Milchmenge ein, weshalb es unerheblich ist, wieviel Luft der Milch beigemischt ist, und welche Personen die Milchförderung vornehmen. Erfindungsgemäß erfolgt die Erfassung des Ist-Volumens der geförderten Milchmenge durch eine kontinuierliche Erfassung des Füllgrades der Milch in der Förderleitung zusätzlich zur kontinuierlichen Erfassung der Fördergeschwindigkeit in dieser Leitung. Über den Füllgrad wird die unterschiedliche Konsistenz der strömenden Milch bei der Probenentnahme in einer Rohrleitung berücksichtigt. Es wird sowohl eine beispielsweise das gesamte Innenrohr abdeckende Wand strömung als auch eine unterschiedliche Lufteinschlüsse aufweisende, aufgeschäumte Milchströmung und ebenso eine bodenbedeckende Strömung erfaßt. Erfindungsgemäß werden der Füllgrad und somit auch der Füllstand sowie die Fördergeschwindigkeit jeweils mit flächigen Fühlorganen, beispielsweise rechteckigen oder quadratischen Elektroden, erfaßt. Um auch den Milchanteil mit hohem Rahmgehalt bei der Probennahme vollständig zu berücksichtigen, wird erfindungsgemäß eine Probenentnahme im Bereich der Sohle der Förderleitung, jedoch mit Abstand zu dieser, durchgeführt. Indem die Probenentnahme mit Abstand zur Sohle der Förderleitung erfolgt, ist gewährleistet, daß Restmilch des Vorlieferanten nicht erfaßt wird. Dies geschieht vorzugsweise durch Zeitsteuerung, indem erst ab einem vorgegebenen Füllgrad, der aktuell jeweils vom Füllgradsensor ermittelt wird, nach Ablauf eines vorgegebenen Zeitraums die Probennahme freigegeben wird.

Eine repräsentative Milchprobe ist demnach auch unter ungünstigsten Bedingungen in der Praxis sichergestellt, und zwar auch bei Verwendung von Peristaltikpumpen für die Probenehmer-Einrichtung oder anderer impulsgesteuerter oder weitgehend kontinuierlich betriebener, aber durch unterschiedliches Ansaugvermögen gekennzeichneter Probepumpen.

Volumenmessungen sind bekanntlich abhängig von unterschiedlichsten Parametern, wie beispielsweise der Temperatur. Diese Leitwerte werden gemäß einer vorteilhaften Ausgestaltung der Erfindung dadurch einfach und effektiv berücksichtigt, daß zu einem vorgegebenen Zeitpunkt der Entnahme bei vollständig gefüllter Leitung eine Kalibrierung des erfaßten Volumens, insbesondere auf die Temperaturabhängigkeit des Leitwertes der Milch erfolgt. Eine entsprechende automatische Kalibrierungsroutine (Autocalroutine) erfolgt zeitgesteuert während des Absaugens beim Lieferanten.

Um Verzugszeiten beim Erfassen des Milchvolumens zu vermeiden, ist es vorteilhafterweise vorgesehen, den Abstand zwischen Probeentnahmeort und Meßort für das momentane Ist-Volumen der geförderten Milchmenge in Gestalt einer Verzögerungskonstante zu berücksichtigen.

Den Erfordernissen der Praxis wird ferner erfindungsgemäß dadurch Rechnung getragen, daß zu Beginn der Milchabsaugung vorgegebene Daten, wie beispielsweise die aus den Vortagen ermittelte Saugleistung, bei der Probenahme berücksichtigt werden. Unverzüglich nach Beginn der Erfassung des momentanen Ist-Volumens der Milchcharge bzw. Milchmenge wird dieser vorgegebene Wert dann durch die aktuellen Daten ersetzt.

Um Produktionsvarianten beim Lieferanten, nämlich unterschiedliche Anliefermengen der Milch, berücksichtigen zu können, ist es vorteilhafterweise vorgesehen, der Milchmenge entsprechende vorausgehende Daten, wie beispielsweise die Daten vom Vortag, mit einer vorgegebenen Toleranz zu berücksichtigen. Sind beispielsweise am Vortag insgesamt 100 1 Milch bei einem bestimmten Lieferanten abgesaugt worden, so werden aufgrund der Toleranz am nächsten Tag beispielsweise bis etwa 120 1 zugelassen, ohne daß diese Übermenge zu einer ungültigen Probenahme führt.

Das Problem Restmilch kann auch dadurch vermieden werden, daß beim Abschluß einer Probenahme und damit beim Abschluß des Absaugens aus einer Kanne vom Lieferanten ein kurzes Rückspülen über die Pumpe der Probenehmer-Einrichtung erfolgt, so daß die Schlauchleitung für die Probenahme frei von Restmilch ist. Auf einen entsprechenden Vorspülvorgang, bei dem zunächst über die Probeleitung ganz kurz eine Teilprobe angesaugt wird, ohne daß diese in die Probenehmerflasche gelangt, kann zumindest dann verzichtet werden, wenn kleine Absaugmengen anstehen.

Die Vorrichtung, die insbesondere zur Durchführung des erfindungsgemäßen Verfahrens jedoch auch für eine präzise Steuerung bei anderen Flüssigprobenentnahmen geeignet ist und einen Sensor zur Erfassung der Milchfördergeschwindigkeit und einen Sensor zur Erfassung der Leitfähigkeit der Milchcharge, eine Datenerfassungseinrichtung und eine mit dieser verbundenen Steuereinrichtung zur Steuerung einer Pumpe für die Probenentnahme in Abhängigkeit von den erfaßten Daten aufweist, ist zusätzlich zu dem Sensor zur Erfassung der Milchfördergeschwindigkeit mit einem ebenfalls im Ansaugweg der Förderleitung angeordneten Leitfähigkeitssensor zur Erfassung des Füllstandes und/oder des Füllgrades versehen.

In einer vorteilhaften Weise ist der Sensor zur Ermittlung der Fördergeschwindigkeit ebenso wie der Leitfähigkeitssensor zur Füllgradermittlung ein mechanikfreier Sensor, vor allem ein Sensor ohne bewegliche Fühlorgane. Dadurch wird erreicht, daß die Meßwerte von den Sensoren selbst nicht ungünstig beeinflußt werden. Als Sensor zur Ermittlung der Fördergeschwindigkeit ist ein magnetisch induktiver Durchflußmesser bevorzugt vorzusehen. Zur nahezu fehlerfreien Erfassung des momentanen Ist-Volumens der Milchfördermenge sind sowohl der Sensor zur Erfassung der Fördergeschwindigkeit als auch der Sensor zur Erfassung des Füllgrades als flächige Fühlorgane, insbesondere als rechteckige oder quadratische Elektroden, ausgebildet.

Es ist zweckmäßig, die Fühlorgane derart in einem entsprechend ausgebildeten Meßraum anzuordnen, daß der gesamte Raum und damit das gesamte Volumen von der Messung erfaßbar ist. Bevorzugt weist der Meßraum einen rechteckigen oder quadratischen Querschnitt auf. Die Geometrie des Meßraumes gewährleistet in Verbindung mit der Elektrodengeometrie die angestrebte präzise Erfassung des Milchmengen-Ist-Volumens.

Die erfindungsgemäße Vorrichtung weist außerdem eine mit ihrer Öffnung nahe über der Sohle der Förderleitung angeordnete Entnahmeeinrichtung auf, insbesondere eine Absaugkanüle mit einer gegen die Förderrichtung geneigten Öffnung.

Beide Sensoren sind in Bezug auf die Förderrichtung vorzugsweise hintereinander angeordnet, wobei die Reihenfolge der Sensoren grundsätzlich beliebig ist. Es ist zweckmäßig, beide Sensoren in einem gemeinsamen Gehäuse zu integrieren und auch die Elektroden dieser Sensoren in einem gemeinsamen Meßraum anzuordnen. Zur exakten Erfassung des Füllgrades ist es sinnvoll, die flächigen Elektroden innerhalb des Meßraumes in einer senkrechten Anordnung zu fixieren.
Im Hinblick auf eine fehlerfreie Kalibrierung ist es vorteilhaft, zusätzliche Elektroden am unteren und oberen Innenrohr vorzusehen, um damit die Bodenbedeckung der Leitung und die vollständige Füllung über den gesamten Querschnitt ermitteln zu können.

Bei anderen als rechteckigen Leitungsquerschnitten kann es zweckmäßig sein, die Geometrie der Elektroden abhängig vom Leitungsquerschnitt anzupassen, um damit eine präzise, unverfälschte Meßwerterfassung zu erreichen. Beispielsweise empfiehlt es sich die Elektroden bei einem runden Leitungsquerschnitt im mittleren Bereich mit einer Einschnürung auszulegen, um damit die präzise Meßwerterfassung zu erreichen.

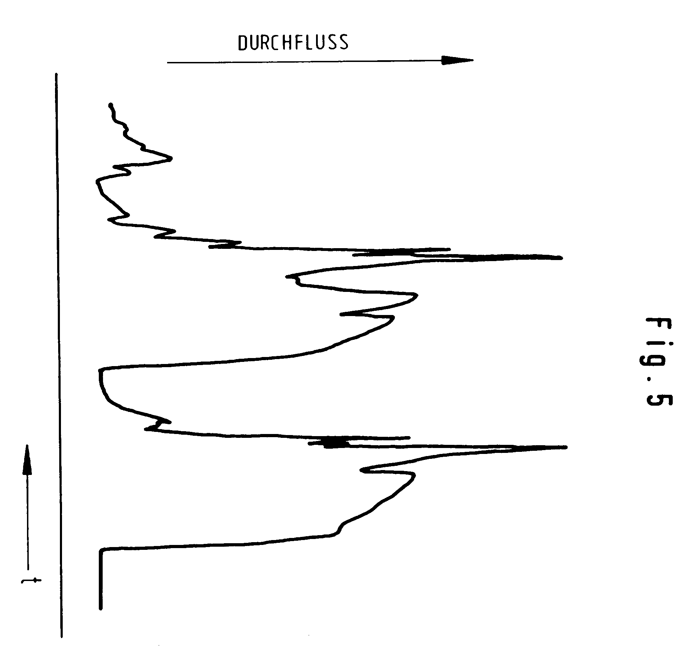

Bei dieser geometrischen Anpassung der Elektroden wird die im wesentlichen senkrechte Anordnung der Elektroden (Fig. 4) angenommen.

Nachfolgend wird die Erfindung anhand einer Zeichnung weiter beschrieben; in dieser zeigen:
- Fig. 1: eine schematische Ansicht einer Anordnung zum Absaugen von Milch mit der erfindungsgemäßen Vorrichtung zur Entnahme einer repräsentativen Milchprobe;
- Fig. 2: eine Draufsicht auf die Anordnung von Fig. 1;
- Fig. 3: einen vertikalen Längsschnitt durch eine Förderrohrleitung im Bereich der Sensoren;
- Fig. 4: einen Querschnitt nach Linie IV-IV in Fig. 3 und
- Fig. 5: ein Geschwindigkeitsprofil einer mit der Anordnung von Fig. 1 aus zwei z.B. 40 l-Behältern geförderten Milchcharge.

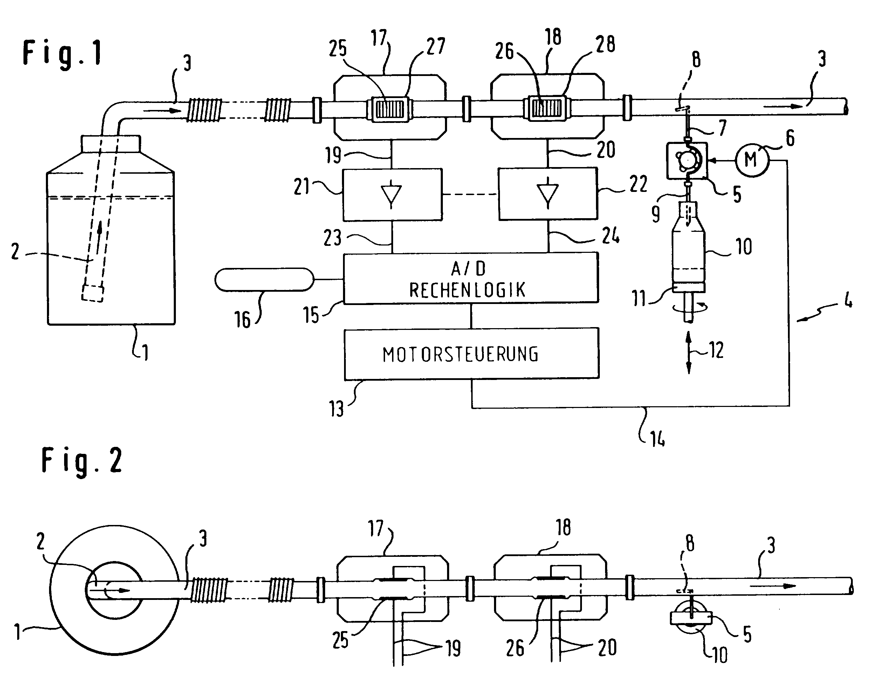

Die in Fig. 1 gezeigte Milchförderanordnung umfaßt einen Behälter 1 mit von einem Lieferanten bereitgestellter Milch und eine, beispielsweise an ein Milchsammelfahrzeug angeordnete Einrichtung zur Milchabsaugung sowie zur Probenahme, wobei ein Behältnis auf dem Milchsammelfahrzeug zur Übernahme der Milch aus dem Behälter 1 nicht dargestellt ist.

In den Behälter 1 ragt der Krümmer 2 einer Förderrohrleitung 3, deren anderes Ende an dem Vorratsbehälter oder den Vorratsbehältern des Milchsammelfahrzeugs angeschlossen ist. Mit dem Ansaugtrakt der Förderleitung 3 steht eine Probenehmer-Einrichtung 4 in Übertragungsverbindung, die zur repräsentativen Milchprobenahme ausgelegt ist und eine Schlauchpumpe 5 umfaßt, die über einen Elektromotor 6 angetrieben wird. Diese Pumpe 5 steht am Ansaugende über eine Rohrleitung 7 mit einer Ansaugkanüle 8 in Verbindung, die in die Förderleitung 3 hineinragt. Am anderen Ende mündet eine Schlauchleitung 9 in einen Probenahmebehälter 10 hinein. Der Behälter 10 ist auf einem Träger 11 angeordnet, der in Richtung des Doppelpfeils 12 auf und ab fahrbar ist, um eine Probeflasche 10 in Gegenüberlage zu der Leitung 9 zu bringen, bzw. die Flasche 10 aus dieser Position abzusenken und an eine nicht dargestellte Fördereinrichtung abzugeben. Mit dieser wird die gefüllte Probeflasche ab- und eine neue Probeflasche angeliefert. Die Probeflaschen 10 sind vorzugsweise mit einem Stopfen hermetisch verschlossen. Die Schlauchleitung 9 weist am freien Ende eine feststehende Nadel auf, die beim Anheben des Trägers 11 den Stopfen zu durchdringen vermag. Um diesen Vorgang zu unterstützen, ist die Flasche 10 auf dem Träger 11 festgesetzt und der Träger 11 rotiert sowohl bei seiner Aufwärtswie bei seiner Abwärtsbewegung, um den Einstechvorgang der Nadel bzw. das Abziehen der Flasche mit dem Stopfen von der Nadel zu erleichtern.

Die Pumpe 5 der Probenehmer-Einrichtung 4 wird in Abhängigkeit vom momentanen Ist-Volumen der geförderten Milchmenge gesteuert. Hierzu ist eine Steuereinrichtung 13 vorgesehen, die über eine Steuerleitung 14 mit dem Motor 6 der Pumpe 5 in Verbindung steht. Die Motorsteuereinrichtung 13 ist eingangsseitig mit einer Rechenlogik 15 verbunden, die den Teil einer Datenerfassungseinrichtung bildet, über welche unter anderem die Daten des Lieferanten sowie Soll-Daten zur Milchcharge über eine Eingabeeinrichtung, wie beispielsweise über eine Tastatur 16, eingegeben werden können. Der Rechenlogik 15 werden zudem Signale aus Sensoren 17 und 18 zugeführt, die in die Förderleitung 3 eingesetzt sind, und deren Ausgangssignale über Leitungen 19 und 20 in Schaltkreisen 21 und 22 aufbereitet werden, bevor sie über Leitungen 23 und 24 der Rechenlogik 15 zugeführt werden, die eingangsseitig zudem Analog/Digitalwandler zur Umwandlung der aufbereiteten analogen Sensorsignale in digitale Signale umfaßt.

Bei den in der Leitung 3 hintereinander angeordneten Sensoren handelt es sich um einen Sensor 17 zur Erfassung der Fördergeschwindigkeit, der vorzugsweise als magnetisch induktiver Durchflußmesser (MID) ausgelegt ist und um einen Sensor 18 zur Erfassung des Füllgrades in der Förderleitung 3, der als Leitfähigkeitssensor (G-Sensor) ausgelegt ist. Wesentlich für die Genauigkeit der erfaßten Fördergeschwindigkeit und des erfaßten Füllgrades ist es, daß beide Sensoren ohne mechanische Erfassungsorgane ausgebildet sind. Die Sensoren 17, 18 sind insgesamt so angeordnet, daß sie nicht in die Strömung hineinragen und damit die Strömung im Hinblick auf Turbulenzen und dergleichen nicht beeinträchtigen. Insbesondere weisen die Sensoren 17, 18 keine beweglichen Fühlorgane auf.
Ferner ist für die Genauigkeit der Datenerfassung maßgebend, daß beide Sensoren 17 und 18 jeweils flächige Elektroden 25 und 26 zur Meßwerterfassung aufweisen, die in einem rechteckigen oder quadratischen Raum 27 bzw. 28 der Sensorengehäuse oder in oder an den Flächen eines Rechteckquerschnittes der Absaugleitung 3 angeordnet sind.

Die Sensoren 17 und 18 erfassen daher präzis und kontinuierlich den Milchdurchfluß und den Füllgrad sowie Füllstand in der Leitung 3, wobei diese Meßwerte nach Durchlaufen der Signalaufbereitungsstufen 21 und 22 in der Rechenlogik 15 zur Darstellung des momentanen Ist-Volumens der geförderten Milchmenge miteinander verknüpft werden. In Abhängigkeit von dem derart ermittelten momentanen Ist-Volumen der geförderten Milchmenge erfolgt die Ansteuerung des Motors 6 für die Pumpe 5 über die Motorsteuerung 13 unter Berücksichtigung der Daten des Lieferanten und der Soll-Daten zur Milchcharge derart, daß eine repräsentative Milchprobenahme gewährleistet ist. Ein wesentlicher Aspekt besteht dabei darin, daß die Milchprobenahme ausschließlich in Abhängigkeit von dem geförderten Ist-Volumen in der Milchmenge erfolgt, wodurch beispielsweise in der Milch enthaltene Luft die Probenahme nicht ungünstig beeinflussen kann. Abhängigkeiten von Störfaktoren, wie beispielsweise in der Milch enthaltener Luft oder einer Wandströmung, die bei einer Füllstandsmessung zu verfälschten Meßergebnissen führt, werden daher eliminiert.

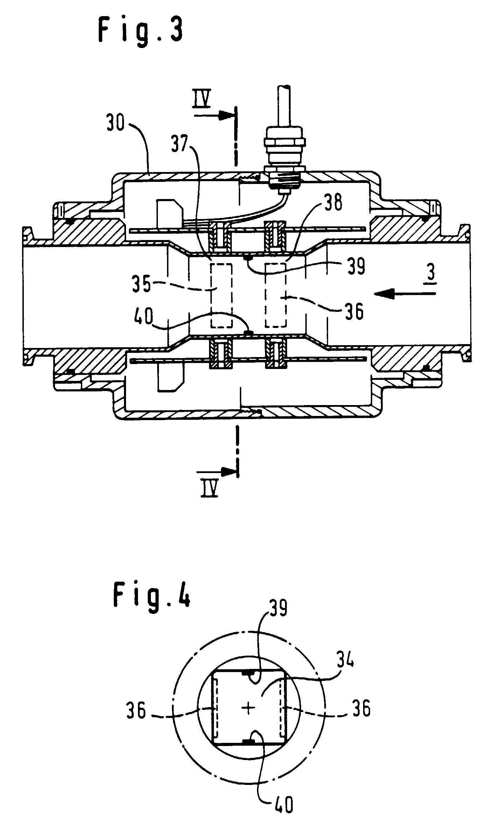

In Fig. 3 und 4 sind in einer alternativen Anordnung ein MID 37 als Durchflußmesser und ein Leitfähigkeitssensor 38 zur Erfassung des Füllgrades in einem gemeinsamen Gehäuse 30 angeordnet. Als flächige Fühlorgane sind rechteckige Elektroden 35,36 eingesetzt, die im Prinzip identisch ausgebildet sind. In einem geringfügig verjüngten Bereich der Förderleitung 3 sind die jeweils parallel angeordneten Elektroden 35 und 36 der beiden Sensoren 37 und 38 derart angeordnet, daß nahezu der gesamte Rohrquerschnitt bei den Messungen erfaßt wird. Diese Sensoren 37, 38 erlauben auch die Erfassung des Füllstandes in der Förderleitung 3. Zur zusätzlichen Sicherheit für die bevorzugt automatische Kalibrierungsroutine sind außerdem in dem nahezu quadratischen Meßraum 34 in einer oberen und unteren mittigen Anordnung zusätzliche, relativ kleinflächige Elektroden 39, 40 vorgesehen, die insbesondere Daten zur Bodenbedeckung und einem Durchfluß über den vollen Querschnitt der Leitung liefern. Bei diesem Ausführungsbeispiel sind die Elektroden 36 des Leitfähigkeitssensors 38 zur Bestimmung des Füllgrades in Flußrichtung vor dem MID 37 angeordnet.

Fig. 5 zeigt ein Geschwindigkeitsprofil der in der Förderleitung geförderten Milch, wobei die Förderung nacheinander aus zwei Behältern 1 erfolgt. Die Zeitachse verläuft in der Fig. 5 auf der Abszisse von rechts nach links, und der erste Impulszug entspricht dem Geschwindigkeitsprofil bei der Milchförderung aus dem ersten Behälter und der zweite Impulszug dem Geschwindigkeitsprofil bei der Förderung aus dem zweiten Behälter. Dabei wird deutlich, daß zunächst ein steiler Anstieg des Absaugvolumens erfolgt, der im Laufe der Förderung flacher verläuft, wobei Einbrüche im Fördervolumen das Vorhandensein von Luft in der Milch zeigen, die von dem Füllgradsensor erfaßt werden.

Wie aus Fig. 1 ersichtlich, verläuft der Endabschnitt der Kanüle 8 geneigt entgegengesetzt zu der Milchförderrichtung. Dieser Kanülenabschnitt endet mit seiner Öffnung in einem Abstand zu der Sohle der Leitung 3. Durch diese Abwinkelung der Entnahmekanüle 8 wird erreicht, daß auch bei relativ niedrigstem Stand der abgesaugten Milch im Rohr noch eine Probe genommen werden kann. Andererseits liegt die Absaugkanüle mit ihrer Öffnung soweit über dem untersten Totpunkt des Rohres, daß in der Leitung 3 vorhandene Restmilch eines vorausgegangenen Lieferanten nicht für die repräsentative Probe des nachfolgenden Lieferanten berücksichtigt wird. Aufgrund der insbesondere geneigten Anordnung der Kanüle 8 und der großflächigen Ausbildung der Elektroden der Sensoren zur Erfassung der Fördergeschwindigkeit und des Füllgrades wird außerdem erreicht, daß auch fettreiche oder rahmreiche Milch repräsentativ erfaßt werden kann.

## Patentansprüche

1. Verfahren zur Entnahme einer repräsentativen Milchprobe bei der Förderung einer Milchcharge eines Lieferanten aus einem Behälter über eine Förderleitung in einen Sammelbehälter, bei dem Daten des Lieferanten und Soll-Daten zur Milchcharge sowie fortlaufend erfaßte Daten über die Fördergeschwindigkeit und die Füllung der Förderleitung in eine Datenerfassungseinrichtung eingegeben und diese Daten einer Steuereinrichtung zugeführt werden, die eine Probenehmer-Einrichtung zur Entnahme der repräsentativen Milchprobe mittels einer Pumpe, insbesondere einer Peristaltikpumpe, aus der Förderleitung unter Berücksichtigung dieser Daten und gespeicherter Parameter steuert,
dadurch **gekennzeichnet**,
daß die Steuerung der Pumpe der Probenehmer-Einrichtung in Abhängigkeit vom momentanen Ist-Volumen der geförderten Milchmenge erfolgt,
daß das Ist-Volumen aus den im Ansaugweg der Förderleitung fortlaufend erfaßten Daten über die Fördergeschwindigkeit und aus fortlaufend erfaßten Daten über den Füllgrad der Förderleitung ermittelt wird,
daß die Fördergeschwindigkeit und der Füllgrad jeweils mit flächigen Fühlorganen erfaßt wird und
daß die Probenentnahme im Bereich der Sohle der Förderleitung, jedoch unter Abstand zu dieser, erfolgt.

2. Verfahren nach Anspruch 1,
dadurch **gekennzeichnet**,
daß zu einem vorgegebenen Zeitpunkt der Entnahme bei vollständig gefüllter Leitung eine Kalibrierung des erfaßten Milchmengen-Volumens erfolgt.

3. Verfahren nach Anspruch 1 oder 2,
dadurch **gekennzeichnet**,
daß zusätzlich der Füllstand erfaßt wird, daß die Erfassung der Fördergeschwindigkeit, des Füllgrades und des Füllstandes an einem dem Probenahmeort vorgeschalteten Meßort erfolgt, und daß der Abstand zwischen Probenahme und Meßort bei der Steuerung der Pumpe der Probenehmer-Einrichtung in Gestalt einer Verzögerungskonstante, die gegebenenfalls von der Durchflußgeschwindigkeit abgeleitet wird, berücksichtigt wird.

4. Verfahren nach einem der Ansprüche 1 bis 3,
dadurch **gekennzeichnet**,
daß die Pumpe der Probenehmer-Einrichtung im Zeitraum vom Beginn der Milchchargen-Förderung bis zur Ermittlung des ersten Ist-Volumenwerts der geförderten Milchmenge mit einer vorgegebenen Leistung betrieben wird.

5. Vorrichtung zur Entnahme einer repräsentativen Milchprobe bei der Förderung einer Milchcharge eines Lieferanten aus einem Behälter (1) über eine Förderleitung (3) in einen Sammelbehälter, mit einem Sensor (17) zur Erfassung der Milchfördergeschwindigkeit und einem Sensor (18) zur Erfassung der Leitfähigkeit der Milchcharge, mit einer Datenerfassungseinrichtung (15) für die Soll-Daten zur Milchcharge und zur Eingabe mindestens von Ist-Daten des Lieferanten sowie für fortlaufend erfaßte Daten über die Fördergeschwindigkeit und mit einer mit der Datenerfassungseinrichtung (15) verbundenen Steuereinrichtung (13) zur Steuerung einer mit einer Pumpe (5), insbesondere einer Peristaltikpumpe, ausgestatteten Probenehmereinrichtung (4) für die Entnahme der repräsentativen Milchprobe in Abhängigkeit von den erfaßten Daten, insbesondere zur Durchführung des Verfahrens nach einem der Ansprüche 1 bis 4,
dadurch **gekennzeichnet**,
daß der im Ansaugweg der Förderleitung (3) zusätzlich zu dem Sensor (17) zur Erfassung der Milchfördergeschwindigkeit angeordnete Leitfähigkeitssensor (18) zur Erfassung des Füllgrades vorgesehen ist,
daß die Sensoren (17,18) flächige Fühlorgane (25,26) aufweisen,
daß die Datenerfassungseinrichtung (15) dazu ausgelegt ist, das momentane Ist-Volumen der geförderten Milchmenge aus den fortlaufend erfaßten Daten über die Fördergeschwindigkeit und den Füllgrad zu ermitteln und der Steuereinrichtung (13) zur Steuerung der Pumpe (5,6) der Probenehmereinrichtung (4) zuzuführen und
daß die Probenehmereinrichtung (4) eine mit ihrer Öffnung nahe über der Sohle der Förderleitung (3) angeordnete Entnahmeeinrichtung aufweist.

6. Vorrichtung nach Anspruch 5,
dadurch **gekennzeichnet**,
daß die Sensoren (17,18) zur Erfassung der Fördergeschwindigkeit und des Füllgrades mechanikfreie Sensoren bzw. Sensoren ohne bewegliche Fühlorgane sind und/oder daß als Fühlorgane (25,26) rechteckige Elektroden in einem etwa rechteckigen Innenraum (27,28) angeordent sind.

7. Vorrichtung nach einem der Ansprüche 5 oder 6,
dadurch **gekennzeichnet**,
daß der Sensor zur Erfassung der Fördergeschwindigkeit (37) und der Sensor zur Erfassung des Füllgrades (38) in einem gemeinsamen Gehäuse (30) angeordnet sind und daß zwischen den flächigen Elektroden (35,36) der Sensoren (37,38) zusätzliche Elektroden (39,40) in einer oberen und unteren mittigen Anordnung vorgesehen sind.

8. Vorrichtung nach Anspruch 5,
dadurch **gekennzeichnet**,
daß als Entnahmeeinrichtung (8) eine Kanüle mit einem die Kanülenöffnung aufweisenden Abschnitt vorgesehen ist, welcher gegen die Förderrichtung geneigt oder gerade angeordnet ist.

9. Vorrichtung nach einem der vorhergehenden Ansprüche 5 bis 8,
dadurch **gekennzeichnet**,
daß der Sensor zur Erfassung der Fördergeschwindigkeit (17,37) ein magnetisch induktiver Durchflußmesser und der Sensor zur Erfassung des Füllgrades (18,38) ein Leitfähigkeitssensor ist, welche in Förderrichtung hintereinander angeordnet sind.

10. Vorrichtung nach Anspruch 9,
dadurch **gekennzeichnet**,
daß der Leitfähigkeitssensor (38) in Förderrichtung vor dem magnetisch induktiven Durchflußmesser (37) angeordnet ist.

## Claims

1. Process for taking a representative milk sample during the delivery of a milk charge of a supplier from a tank via a delivery line into a collecting tank, in which the supplier data and desired milk charge data as well as continuously detected data concerning the delivery rate and the filling of said delivery line are inputted into a data acquisition means and said data are supplied to a control means, which controls a sampling means for taking said representative milk sample by means of a pump, in particular a peristaltic pump, out of said delivery line, while taking into account said data and stored parameters,
**characterized** in that
control of said pump of said sampling means is carried out as a function of the momentary actual volume of the delivered milk quantity,
the actual volume is determined by means of said data concerning said delivery rate continuously detected in the intake portion of said delivery line and by means of said continuously detected data concerning said degree of filling of said delivery line,
said delivery rate and said degree of filling are each detected by means of plane sensing organs, and
in that sampling takes place in the vicinity of the delivery line bottom, however, spaced from the latter.

2. Process according to claim 1,
**characterized** in that
a calibration of the detected milk quantity volume takes place at a predetermined moment during removal when said line is completely filled.

3. Process according to claim 1 or 2,
**characterized** in that
the filling level is additionally detected,
the detection of said delivery rate, said degree of filling and said filling level takes place at a measuring place positioned upstream to the sampling place, and
in that in the controlling of said pump of said sampling means the distance between said sampling and measuring place is taken into consideration as a retardation constant, which is appropriately derived from the flow rate.

4. Process according to one of the claims 1 to 3,
**characterized** in that
said pump of said sampling means is operated with a predetermined output during the period of time from the beginning of said milk charge delivery till the detection of the first actual-volume value of the delivered milk quantity.

5. Device for taking a representative milk sample during the delivery of a milk charge of a supplier from a tank (1) via a delivery line (3) into a collecting tank, with a sensor (17) for detecting the milk delivery rate and a sensor (18) for detecting the conductivity of the milk charge, with a data acquisition means (15) for the desired milk charge data and for inputting at least actual data of said supplier as well as for continuously detected data concerning said delivery rate and with a control means (13) connected to the data acquisition means (15) for controlling a sampling means (4) provided with a pump (5), in particular a peristaltic pump, for taking said representative milk sample as a function of said detected data, in particular for performing the process according to one of the claims 1 to 4,
**characterized** in that
said conductivity sensor (18) arranged in the intake portion of said delivery line (3), in addition to said sensor (17) for detecting said milk delivery rate, is provided for detecting said degree of filling,
said sensors (17, 18) have plane sensing organs (25, 26),
said data acquisition means (15) is designed to detect the actual volume of the delivered amount of milk from said continuously detected data about said delivery rate and said degree of filling and to supply it to said control means (13) for the control of said pump (5, 6) of said sampling means (4), and
in that said sampling means (4) has a removal means, the opening of which is arranged closely above the bottom of said delivery line (3).

6. Device according to claim 5,
**characterized** in that
said sensors (17, 18) for detecting said delivery rate and said degree of filling are mechanic-free sensors respectively sensors without movable sensing organs, and/or
in that rectangular electrodes are arranged as sensing organs (25, 26) in a roughly rectangular interior chamber (27, 28).

7. Device according to claims 5 or 6,
**characterized** in that
the sensor for detecting said delivery rate (37) and the sensor for detecting said degree of filling (38) are arranged in a common casing (30), and
in that additional electrodes (39, 40) are provided in an upper and lower centrical arrangement between said plane electrodes (35, 36) of said sensors (37, 38).

8. Device according to claim 5,
**characterized** in that
as removal means (8) is provided a cannula with a portion having the cannula opening, which portion is either inclined towards the delivery direction or is straight.

9. Device according to one of the preceding claims 5 to 8,
**characterized** in that
the sensor for detecting the delivery rate (17, 37) is a magnetic-inductive flow meter and the sensor for detecting the degree of filling (18, 38) is a conductivity sensor, wherein both sensors are subsequently arranged in delivery direction.

10. Device according to claim 9,
**characterized** in that
said conductivity sensor (38) is arranged in delivery direction upstream to said magnetic-inductive flow meter (37).

## Revendications

1. Procédé de prélèvement d'un échantillon représentatif de lait lors de l'acheminement d'une charge de lait d'un fournisseur depuis un récipient, par l'intermédiaire d'une conduite d'acheminement, vers un récipient de collecte, dans lequel des données du fournisseur et des données de consigne concernant la charge de lait ainsi que des données prélevées en continu sur la vitesse d'acheminement et le remplissage de la conduite d'acheminement sont entrées dans un dispositif de collecte de données et ces données sont transmises à un dispositif de commande, qui commande un dispositif de prise d'échantillon destiné à prélever l'échantillon représentatif de lait au moyen d'une pompe, notamment d'une pompe péristaltique, depuis la conduite d'acheminement en tenant compte de ces données et de paramètres mémorisés,
***caractérisé en ce que*** la commande de la pompe du dispositif de prise d'échantillon se fait en fonction du volume réel momentané de la quantité de lait acheminée,
***en ce que*** le volume réel est déterminé à partir des données sur la vitesse d'acheminement déterminées en continu sur la voie d'aspiration de la conduite et à partir de données déterminées en continu sur le degré de remplissage de la conduite d'acheminement,
***en ce que*** la vitesse d'acheminement et le degré de remplissage sont déterminés chacun par des organes détecteurs plats et
***en ce que*** le prélèvement d'échantillon s'effectue dans la zone de la base de la conduite d'acheminement, mais à distance de celle-ci.

2. Procédé selon la Revendication 1, ***caractérisé en ce qu***' à un instant déterminé du prélèvement, la conduite étant complètement remplie, il est procédé à un calibrage du volume de lait prélevé.

3. Procédé selon la Revendication 1 ou 2, ***caractérisé en ce qu***' additionnellement, le degré de remplissage est déterminé, ***en ce que*** la détermination de la vitesse d'acheminement, du degré de remplissage et du niveau de remplissage se fait en un point de mesure placé avant le point de prélèvement, et ***en ce que*** la distance entre point de prélèvement et point de mesure est prise en compte pour la commande de la pompe du dispositif de prise d'échantillon, sous la forme d'une constante de retard, qui est dérivée le cas échéant de la vitesse d'écoulement.

4. Procédé selon l'une des Revendications 1 à 3, ***caractérisé en ce que*** la pompe du dispositif de prise d'échantillon est actionnée à une puissance prédéterminée pendant la période précédant le début de l'acheminement de la charge de lait et allant jusqu'à la détermination de la première valeur de volume réel de la quantité de lait acheminée.

5. Dispositif de prélèvement d'un échantillon représentatif de lait lors de l'acheminement d'une charge de lait d'un fournisseur depuis un récipient (1), par l'intermédiaire d'une conduite d'acheminement (3), vers un récipient de collecte, avec un capteur (17) pour déterminer la vitesse d'acheminement du lait et un capteur (18) pour déterminer la conductivité de la charge de lait, avec un dispositif de collecte de données (15) pour les données de consigne de la charge de lait et pour entrer au moins des données réelles du fournisseur, ainsi que pour des données déterminées en continu sur la vitesse d'acheminement, et avec un dispositif de commande (13) relié au dispositif de collecte de donnés (15) pour commander un dispositif de prise d'échantillon (4) équipé d'une pompe (5), en particulier une pompe péristaltique, pour prélever l'échantillon représentatif de lait en fonction des données déterminées, en particulier pour exécuter le procédé selon l'une des Revendications 1 à 4,
***caractérisé en ce que*** le capteur de conductivité (18) placé sur la voie d'aspiration de la conduite d'acheminement (3) en plus du capteur (17) de détermination de la vitesse d'acheminement du lait est prévu pour déterminer le degré de remplissage,
***en ce que*** les capteurs (17, 18) présentent des organes détecteurs plats (25, 26),
***en ce que*** le dispositif de collecte de données (15) est conçu pour déterminer le volume réel momentané de la quantité de lait acheminée à partir des données déterminées en continu et du degré de remplissage et pour l'envoyer au dispositif de commande (13) destiné à commander la pompe (5, 6) du dispositif de prise d'échantillon (4), et
***en ce que*** le dispositif de prise d'échantillon (4) présente un dispositif de prélèvement placé avec son ouverture proche et au dessus de la base de la conduite d'acheminement (3).

6. Dispositif selon la Revendication 5, ***caractérisé en ce que*** les capteurs (17, 18) de détermination de la vitesse d'acheminement et le degré de remplissage sont des capteurs sans mécanique ou des capteurs sans organes détecteurs mobiles, et/ou ***en ce que,*** pour les organes détecteurs (25, 26), des électrodes rectangulaires sont placées dans un espace intérieur (27, 28) approximativement rectangulaire.

7. Dispositif selon l'une des Revendications 5 ou 6, ***caractérisé en ce que*** le capteur de détermination de la vitesse d'acheminement (37) et le capteur de détermination du degré de remplissage (38) sont placés dans un boîtier commun (30) et ***en ce qu'*** entre les électrodes plates (35, 36) des capteurs (37, 38), des électrodes supplémentaires (39, 40) sont prévues en des positions centrales supérieure et inférieure.

8. Dispositif selon la Revendication 5, ***caractérisé en ce que,*** comme dispositif de prélèvement (8), il est prévu une canule avec une partie présentant l'ouverture de canule qui est inclinée en sens inverse de la direction d'acheminement ou est disposée droite.

9. Dispositif selon l'une des Revendications 5 à 8, ***caractérisé en ce que*** le capteur de détermination de la vitesse d'acheminement (17, 37) est un débitmètre à induction magnétique et le capteur de détermination du degré de remplissage (18, 38) est un capteur de conductivité, qui sont placés l'un derrière l'autre dans la direction d'acheminement.

10. Dispositif selon la Revendication 9, ***caractérisé en ce que*** le capteur de conductivité (38) est placé, dans la direction d'acheminement, avant le débitmètre à induction magnétique (37).
